# EUROPÄISCHE PATENTSCHRIFT

(11) **EP 3 790 630 B1**
(45) Veröffentlichungstag und Bekanntmachung des Hinweises auf die Patenterteilung: **15.10.2025**
(21) Anmeldenummer: 19712981.0
(22) Anmeldetag: 21.03.2019
(51) Int. Cl.: A61Q 5/10, A61K 8/58, A61K 8/81

(54) **MITTEL ZUM FÄRBEN VON HAAREN ENTHALTEND EIN FILMBILDENDES, HYDROPHILES POLYMER**
HAIR DYEING COMPOSITION COMPRISING A FILM-FORMING HYDROPHILIC POLYMER
COMPOSITION DE TEINTURE CAPILLAIRE COMPRENANT UN POLYMÈRE HYDROPHILE FILMOGÈNE

(30) Priorität: 07.05.2018 DE 102018207025
(43) Veröffentlichungstag der Anmeldung: 17.03.2021
(73) Patentinhaber: Henkel AG & Co. KGaA, 40589 Düsseldorf (DE)
(72) Erfinder: LECHNER, Torsten, 40764 Langenfeld (DE); NOWOTTNY, Marc, 41069 Mönchengladbach (DE); SCHOEPGENS, Juergen, 41366 Schwalmtal (DE)
(86) Internationale Anmeldenummer: PCT/EP2019/057027
(87) Internationale Veröffentlichungsnummer: WO 2019/214871

(56) Entgegenhaltungen:
- EP-A1- 1 532 967
- EP-A2- 2 168 633
- DE-A1- 10 200 185

## Beschreibung

Gegenstand der vorliegenden Anmeldung ist ein Mittel zum Färben von keratinischem Material, insbesondere menschlichen Haaren, welches in einem kosmetischen Träger (a) mindestens eine spezielle organische Siliciumverbindung der Formel (I), (b) mindestens eine farbgebende Verbindung aus der Gruppe der Pigmente und (c) mindestens ein filmbildendes, hydrophiles Polymer ausgewählt aus der Gruppe aus Polyvinylpyrrolidon (PVP) und den Copolymeren des Polyvinylpyrrolidons enthält.

Ein weiterer Gegenstand dieser Anmeldung ist eine Mehrkomponenten-Verpackungseinheit (Kit-of-parts) zum Färben von keratinischem Material, insbesondere menschlichen Haaren, welche getrennt voneinander konfektioniert in drei verschiedenen Containern die Mittel (I), (II) und (III) umfasst. Hierbei enthält das Mittel (I) mindestens eine organische Siliciumverbindung (a) der Formel (I), das Mittel (II) enthält Wasser und das Mittel (III) enthält mindestens eine farbgebende Verbindung aus der Gruppe der Pigmente (b) und mindestens ein filmbildendes, hydrophiles Polymer (c) ausgewählt aus der Gruppe aus Polyvinylpyrrolidon (PVP) und den Copolymeren des Polyvinylpyrrolidons.

Ein dritter Gegenstand der vorliegenden Anmeldung ist ein Verfahren zum Färben von keratinischem Material, welches die Anwendung eines Vorbehandlungsmittels (V) und im Anschluss daran die Anwendung eines Färbemittels (F) umfasst. Hierbei enthält das Vorbehandlungsmittel (V) in einem wasserhaltigen kosmetischen Träger mindestens eine spezielle organische Siliciumverbindung (a) der Formel (I). Das Färbemittel (F) ist gekennzeichnet durch seinen Gehalt an mindestens einer farbgebenden Verbindung aus der Gruppe der Pigmente (b) und mindestens einem filmbildenden, hydrophilen Polymer (c) ausgewählt aus der Gruppe aus Polyvinylpyrrolidon (PVP) und den Copolymeren des Polyvinylpyrrolidons.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

DE 10200195 A1 beschreibt eine semipermanente Haarfärbe- bzw. Haartönungs-Zusammensetzung, die einen Direktfarbstoff, ein Kohlenwasserstofföl und ein Polyalkoxylenmodifiziertes Dimethylpolysiloxan enthält.

EP 1532967 A1 adressiert Mittel und Verfahren zur Formung und Pflege der Frisur, welche mindestens ein Gellan-Gum oder ein Derivat hiervon, 0,001 bis 5 Gew.-% mindestens einer festen Komponente und mindestens ein monovalentes Salz enthalten.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Shampoonierungen besonders widerstandsfähig sind.

Bei der Nacharbeitung der Lehre der EP 2168633 B1 wurden deren Formulierungen nachgestellt. Hierbei hat sich gezeigt, dass ein Nachteil dieser Formulierungen in ihrer mangelhaften Lagerstabilität besteht. Mit den Pigmenten und den hydrophoben Polymeren beinhalten die Formulierungen sehr schlecht lösliche Substanzen, die bei Herstellung der Formulierungen zwar kurzzeitig in Dispersion gebracht werden konnten, über längere Lagerzeiträume jedoch konglomerierten, sich absetzten oder von der Wasserphase separierten. Abhängig von der gewählten Einsatzkonzentration an Pigment und hydrophobem Polymer hat es sich auch direkt bei der Herstellung als schwierig erwiesen, die schlecht löslichen Substanzen ausreichend feinverteilt in Dispersion zu bekommen.

Es war die Aufgabe der vorliegenden Erfindung, ein Färbesystem bereitzustellen, dass mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (wie beispielsweise Pigmente und direktziehende Farbstoffe) in extrem dauerhafter Weise auf den Haaren zu fixieren. Hierbei sollte eine ausreichend hohe Lagerstablität der Formulierungen gewährleistet sein. Darüber hinaus sollte auch das Produktionsverfahren der Formulierungen vereinfacht bzw. optimiert werden.

Überraschenderweise hat sich nun herausgestellt, dass die vorgenannte Aufgabe hervorragend gelöst werden kann, wenn keratinische Materialien, insbesondere menschliche Haare, mit einem Mittel gefärbt werden, welches in einem kosmetischen Träger mindestens eine bestimmte organische Siliciumverbindung, mindestens eine farbgebende Verbindung aus der Gruppe der Pigmente und mindestens ein filmbildendes, hydrophiles Polymer ausgewählt aus der Gruppe aus Polyvinylpyrrolidon (PVP) und den Copolymeren des Polyvinylpyrrolidons.enthält.

Ein erster Gegenstand der vorliegenden Erfindung ist daher ein Mittel zum Färben von keratinischem Material, insbesondere menschlichen Haaren, enthaltend in einem kosmetischen Träger
(a) mindestens eine organische Siliciumverbindung der Formel (I),

   R₁R₂N-L-Si(OR₃)ₐ(R₄)_{b} (I),

   wobei
   - R₁, R₂ beide für ein Wasserstoffatom stehen, und
   - L für eine lineare, zweiwertige C₁-C₆-Alkylengruppe steht,
   - R₃ für ein Wasserstoffatom oder eine C₁-C₆-Alkylgruppe steht,
   - R₄ für eine C₁-C₆-Alkylgruppe steht
   - a, für eine ganze Zahl von 1 bis 3 steht, und
   - b für die ganze Zahl 3 - a steht, und,
(b) mindestens eine farbgebende Verbindung aus der Gruppe der Pigmente, und
(c) mindestens ein filmbildendes, hydrophiles Polymer ausgewählt aus der Gruppe aus Polyvinylpyrrolidon (PVP) und den Copolymeren des Polyvinylpyrrolidons.

Im Rahmen der zu dieser Erfindung führenden Arbeiten hat sich herausgestellt, dass die Einarbeitung der erfindungsgemäßen, organischen Siliciumverbindungen (a), der farbgebenden Verbindungen (b) und der filmbildenden, hydrophilen Polymeren (c) in einen kosmetischen Träger zu Formulierungen mit ausgezeichneter Lagerstabilität führte. Ohne auf diese Theorie eingeschränkt zu sein, wird in diesem Zusammenhang vermutet, dass die gut löslichen, filmbildenden Polymere (c) die farbgebenden Verbindungen in Dispersion halten bzw. ihr Absetzen oder Separieren verhindern. Auch die Herstellung der Formulierungen war vereinfacht, da die hydrophilen Polymere infolge ihrer guten Wasserlöslichkeit sehr einfach in Lösung zu bringen waren. Als besonders vorteilhaft hat sich herausgestellt, dass auf diesem Wege auch ohne den Einsatz großer Mengen an Tensiden besonders stabile Formulierungen erzeugt werden konnten.

Überraschenderweise konnte weiterhin gefunden werden, dass trotz Einsatz eines hydrophilen, d.h. eigentlich gut wasserlöslichen Polymers (c) zusammen mit den weiteren Inhaltsstoffen (a) und (b) ein sehr resistenter Film auf dem keratinischen Material erzeugt werden konnte. Aus diesem Grund wurden auf dem keratinischen Material überaus waschechte Färbungen mit guter Resistenz gegenüber Shampoonierungen erhalten.

### Mittel zum Färben von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Die erfindungsgemäßen Mittel enthalten die erfindungswesentlichen Verbindungen (a), (b) und (c) in einem kosmetischen Träger, bevorzugt in einem geeigneten wässrigen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaum-formulierungen oder andere Zubereitungen, die für die Anwendung auf dem Haar geeignet sind.

Der kosmetische Träger ist bevorzugt wasserhaltig, was bedeutet, dass der Träger - bezogen auf sein Gewicht - mindestens 2 Gew.-% Wasser enthält. Bevorzugt liegt der Wassergehalt oberhalb von 5 Gew.-%, weiter bevorzugt oberhalb von 10 Gew.-% noch weiter bevorzugt oberhalb von 15 Gew.-%. Der kosmetische Träger kann auch wässrig-alkoholisch sein. Unter wässrig-alkoholischen Lösungen sind im Sinne der vorliegenden Erfindung wässrige Lösungen enthaltend 2 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Begriff "Mittel zur Färbung" wird im Rahmen dieser Erfindung für eine durch Einsatz von Pigmenten hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich in situ durch Oligomerisierung bzw. Polymerisierung des oder der organischen Siliciumverbindungen, und durch die Wechselwirkung von farbgebender Verbindung und organischer Silicumverbindung mit dem filmbildenden, hydrophilen Polymer.

### Organische Siliciumverbindungen

Als ersten erfindungswesentlichen Bestandteil (a) enthalten die erfindungsgemäßen Mittel mindestens eine organische Siliciumverbindung der Formel (I).

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist.

Die Substituenten R₁, R₂, R₃, R₄, L, in den Verbindungen der Formel (I) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ für ein Wasserstoffatom

Im Mittelteil der organischen Siliciumverbindung befindet sich die Struktureinheit oder der Linker -L-,der für eine lineare zweiwertige C₁-C₆-Alkylengruppe steht. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Das erfindungsgemäße Mittel ist dadurch gekennzeichnet, dass es (a) mindestens eine organische Siliciumverbindung der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht.

Die erfindungsgemäßen organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Färbungen mit den besten Waschechtheiten konnten erhalten werden, wenn das erfindungsgemäße Mittel mindestens eine organische Siliciumverbindung der Formel (I) enthält, in welcher die Reste R₃, R₄ unabhängig voneinander für eine Methylgruppe oder für eine Ethylgruppe stehen.

Weiterhin konnten Färbungen mit den besten Waschechtheiten erhalten werden, wenn das erfindungsgemäße Mittel mindestens eine organische Siliciumverbindung der Formel (I) enthält, in welcher der Rest a für die Zahl 3 steht. In diesem Fall steht der Rest b für die Zahl 0.

In einer weiteren bevorzugten Ausführungsform ist ein erfindungsgemäßes Mittel dadurch gekennzeichnet, dass es (a) mindestens eine organische Siliciumverbindung der Formel (I) enthält, wobei
- R₃, R₄ unabhängig voneinander für eine Methylgruppe oder für eine Ethylgruppe stehen und
- a für die Zahl 3 steht und
- b für die Zahl 0 steht.

Zur Lösung der erfindungsgemäßen Aufgabenstellung besonders gut geeignete organische Silicumverbindungen der Formel (I) sind
- (3-Aminopropyl)triethoxysilan 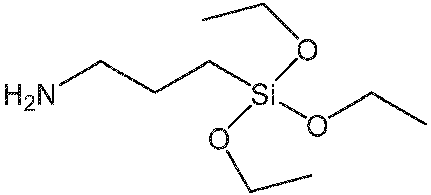
- (3-Aminopropyl)trimethoxysilan 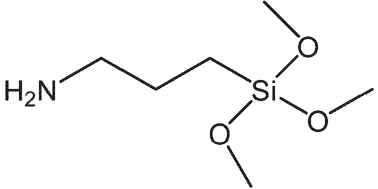
- 1-(3-Aminopropyl)silantriol 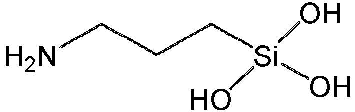
- (2-Aminoethyl)triethoxysilan 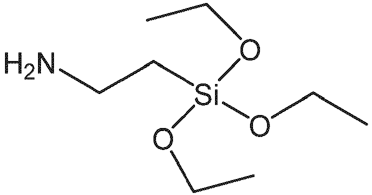
- (2-Aminoethyl)trimethoxysilan 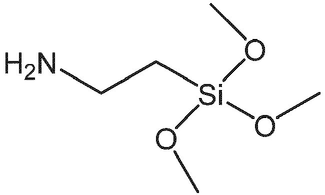
- 1-(2-Aminoethyl)silantriol

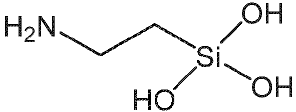

In einer weiteren bevorzugten Ausführungsform ist ein erfindungsgemäßes Mittel dadurch gekennzeichnet, dass es (a) mindestens eine organische Siliciumverbindung der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Bei den organische Siliciumverbindungen der Formel (I) handelt es sich um reaktive Verbindungen. In diesem Zusammenhang hat es sich als bevorzugt herausgestellt, wenn das erfindungsgemäße Mittel - bezogen auf sein Gesamtgewicht - eine oder mehrere organische Siliciumverbindungen (a) in einer Gesamtmenge von 0,1 bis 20,0 Gew.-%, bevorzugt 0,2 bis 15,0 Gew.-% und besonders bevorzugt 0,2 bis 2,0 Gew.-% enthält.

In diesem Zusammenhang hat es sich als besonders bevorzugt herausgestellt, wenn das erfindungsgemäße Mittel - bezogen auf sein Gesamtgewicht - eine oder mehrere organische Siliciumverbindungen (a) der Formel (I) in einer Gesamtmenge von 0,1 bis 20,0 Gew.-%, bevorzugt 0,2 bis 15,0 Gew.-% und besonders bevorzugt 0,2 bis 2,0 Gew.-% enthält.

### Farbgebende Verbindungen aus der Gruppe der Pigmente

Als zweiten erfindungswesentlichen Inhaltsstoff (b) enthalten die erfindungsgemäßen Mittel mindestens eine farbgebende Verbindung aus der Gruppe der Pigmente

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es (b) mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch (b) ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Mittel dadurch gekennzeichnet, dass es (b) mindestens eine farbgebende Verbindungen aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Das oder die Pigmente (b) können in einer Menge von 0,001 bis 20 Gew.-%, insbesondere von 0,05 bis 5 Gew.-%, jeweils bezogen auf das Gesamtgewicht des erfindungsgemäßen Mittels, eingesetzt werden.

Direktziehende Farbstoffe besitzen eine Löslichkeit in Wasser (760 mmHg) bei 25 °C von mehr als 0,5 g/L und sind daher nicht als Pigmente anzusehen.

### filmbildendes, hydrophiles Polymer

Als dritten erfindungswesentlichen Inhaltsstoff (c) enthalten die erfindungsgemäßen Mittel mindestens ein filmbildendes, hydrophiles Polymer ausgewählt aus der Gruppe aus Polyvinylpyrrolidon (PVP) und den Copolymeren des Polyvinylpyrrolidons.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers (c) nicht mehr als 10⁷ g/mol, bevorzugt nicht mehr als 10⁶ g/mol und besonders bevorzugt nicht mehr als 10⁵ g/mol beträgt.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint markoskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Unter einem filmbildenden Polymer wird im Sinne der Erfindung ein Polymer verstanden, welches in der Lage ist, auf einem Substrat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Als filmbildende, hydrophile Polymere können nichtionische, anionische und kationische Polymere eingesetzt werden.

Geeignete filmbildende, hydrophile Polymere werden aus der Gruppe der Polyvinylpyrrolidon-(Co)Polymere, ausgewählt.

Polyvinylpyrrolidon als filmbildendes, hydrophiles Polymer (c) ließ sich sehr einfach und leicht in Wasser lösen und hielt auch größere Einsatzmengen an Pigmenten über lange Zeit stabil in Dispersion. Überraschenderweise war auch die Waschechtheit der Färbungen, die mit mit PVPhaltigen Formulierungen erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskol^{®}K 90 oder Luviskol^{®} K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren (c) aus der Gruppe der Copolymere des Polyvinylpyrrolidons hat ebenfalls zu besonders guten und waschechten Farbergebnissen geführt. Auch die Lagerstabilitäten der Formulierungen, die ein oder mehrere Copolymere des Polyvinylpyrrolidons (c) enthielten, waren sehr gut.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons (c) können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein erfindungsgemäßes Mittel dadurch gekennzeichnet, dass es (c) mindestens ein filmbildendes, hydrophiles Polymer enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copoylmeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes Keratinmaterial, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

In einer ganz besonders bevorzugten Ausführungsform ist ein erfindungsgemäßes Mittel dadurch gekennzeichnet, dass es (c) mindestens ein nichtionisches, filmbildendes, hydrophiles Polymer enthält.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C₁ bis C₄)-Alkylamino-(C₂ bis C₄)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches erfindungsgemäßes Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-WasserGemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Das oder die erfindungsgemäßen filmbildenden hydrophilen Polymere (c) werden bevorzugt in bestimmten Mengenbereichen im erfindungsgemäßen Mittel eingesetzt. In diesem Zusammenhang hat es sich zur Lösung der erfindungsgemäßen Aufgabenstellung als besonders bevorzugt erwiesen, wenn das Mittel - bezogen auf sein Gesamtgewicht - ein oder mehrere Polymere in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

In einer weiteren bevorzugten Ausführungsform ist ein erfindungsgemäßes Mittel dadurch gekennzeichnet, dass es - bezogen auf sein Gesamtgewicht - ein oder mehrere filmbildende hydrophile Polymere (c) in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

### Wassergehalt der Mittel

Das erfindungsgemäße Mittel enthält die wesentlichen Inhaltsstoffe (a), (b) und (c) in einem kosmetischen Träger, bevorzugt in einem wässrigen oder wasserhaltigen kosmetischen Träger.

Ohne an diese Theorie gebunden zu sein, wird vermutet, dass die organische Siliciumverbindung (a), die eine oder mehrere Hydroxylgruppen oder hydrolysierbare Gruppen pro Molekül umfasst, in Anwesenheit des Wassers hydrolysiert und/oder oligomerisiert. Die auf diese Weise entstehenden Hydrolyseprodukte bzw. Oligomere besitzen eine besonders hohe Affinität zur Oberfläche des Keatinmaterials. Mit den farbgebenden Verbindungen (b) kann sich auf diese Weise zusammen mit dem filmbildenden, hydrophilen Polymer (c) ein stabiler und resistenter Film ausbilden. Als ganz besonders bevorzugt hat es sich in diesem Zusammenhang erwiesen, wenn das Mittel - bezogen auf sein Gesamtgewicht - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

In einer weiteren explizit ganz besonders bevorzugten Ausführungsform ist ein erfindungsgemäßes Mittel dadurch gekennzeichnet, dass es - bezogen auf sein Gesamtgewicht - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

### Mehrkomponenten-Verpackungseinheit (Kit-of-parts)

Bei dem zuvor beschriebenen Mittel des ersten Erfindungsgegenstands handelt es sich um das anwendungsbereite Färbemittel. Dieses enthält mit der oder den organischen Siliciumverbindungen (a) eine Klasse von reaktiven Verbindungen, die wie zuvor beschriebenen in Anwesenheit von Wasser eine Hydrolyse und/oder Oligomerisierung eingehen können.

Zur Erhöhung der Lagerstabilität wird dieses Mittel dem Anwender bevorzugt in Form einer Mehrkomponenten-Verpackungseinheit (Kit-of-parts) zur Verfügung gestellt. Kurz vor der Anwendung auf dem keratinischen Material kann der Anwender die verschiedenen Komponenten dieser Verpackungseinheit vermischen und auf diese Weise das anwendungsbereite Färbemittel herstellen.

Ein zweiter Gegenstand der vorliegenden Erfindung ist eine Mehrkomponenten-Verpackungseinheit (Kit-of-parts) zum Färben von keratinischem Material, insbesondere menschlichen Haaren, welche getrennt voneinander konfektioniert
- einen ersten Container enthaltend ein kosmetisches Mittel (I) und
- einen zweiten Container enthaltend ein kosmetisches Mittel (II) und
- einen dritten Container enthaltend ein kosmetisches Mittel (III) umfasst,
   wobei
- das Mittel (I) mindestens eine organische Siliciumverbindung (a) enthält, wie sie im Detail bereits bei der Beschreibung des ersten Erfindungsgegenstands offenbart wurde,
- das Mittel (II) Wasser enthält und
- das Mittel (III) mindestens eine farbgebende Verbindung aus der Gruppe der Pigmente (b) und mindestens ein filmbildendes, hydrophiles Polymer (c) enthält, wie sie im Detail bereits bei der Beschreibung des ersten Erfindungsgegenstands offenbart wurden.

In einer besonders bevorzugten Ausführungsform wird das anwendungsbereite Färbemittel durch Vermischen der Mittel (I), (II) und (III) hergestellt. Im Rahmen dieser Ausführungsform werden alle drei Mittel (I), (II) und (III) gleichzeitig auf dem keratinischen Material appliziert.

Beispielsweise kann der Anwender das Mittel (I), welches die organische Siliciumverbindung(en) (a) enthält, zunächst mit dem wasserhaltigen Mittel (II) verrühren oder verschütteln. Danach kann der Anwender das Mittel (III), welches den oder die farbgebenden Verbindungen (b) und das oder die filmbildenden, hydrophilen Polymeren (c) enthält, zu dem Gemisch aus (I) und (II) hinzufügen und alle drei Mittel miteinander vermengen.

In einer weiteren ganz besonders bevorzugten Ausführungsform ist es ebenfalls möglich, die Mittel (I), (II) und (III) sukzessive auf dem keratinischen Material anzuwenden, so dass die Mittel erst auf dem Keratinmaterial miteinander interagieren.

Beispielsweise kann der Anwender das Mittel (I), welches die organische Siliciumverbindung(en) (a) enthält, zunächst mit dem wasserhaltigen Mittel (II) verrühren oder verschütteln. Diese Mischung aus (I) und (II) kann der Anwender nun - entweder direkt nach ihrer Herstellung oder nach einer kurzen Reaktionszeit von 10 Sekunden bis 20 Minuten - auf die Keratinmaterialien applizieren. Danach kann der Anwender nun das Mittel (III), welches die farbgebenden Verbindungen (b) und das filmbildende, hydrophile Polymer (c) enthält, auf dem Keratinmaterial anwenden.

Das Mittel (I) enthält mindestens eine organische Siliciumverbindung (a) der Formel (I), wie sie im Detail bereits bei der Beschreibung des ersten Erfindungsgegenstands offenbart wurde.

Um eine möglichst lagerstabile Formulierung bereitstellen zu können, wird das Mittel (I) selbst bevorzugt wasserarm oder wasserfrei konfektioniert.

In einer bevorzugten Ausführungsform ist eine erfindungsgemäße Mehrkomponenten-Verpackungseinheit (Kit-of-parts) dadurch gekennzeichnet, dass das Mittel (I) - bezogen auf das Gesamtgewicht des Mittels (I) - einen Wassergehalt von weniger als 10 Gew.-%, bevorzugt von weniger als 5 Gew.-%, weiter bevorzugt von weniger als 1 Gew.-%, noch weiter bevorzugt von weniger als 0,1 Gew.-% und ganz besonders bevorzugt von weniger als 0,01 Gew.-% enthält.

Das Mittel (II) enthält Wasser. In einer bevorzugten Ausführungsform ist eine erfindungsgemäße Mehrkomponenten-Verpackungseinheit (Kit-of-parts) dadurch gekennzeichnet, dass das Mittel (II) - bezogen auf das Gesamtgewicht des Mittels (II) - einen Wassergehalt von 15 bis 100 Gew.-%, bevorzugt von 35 bis 100 Gew.-%, weiter bevorzugt von 55 bis 100 Gew.-%, noch weiter bevorzugt von 65 bis 100 Gew.-% und ganz besonders bevorzugt von 75 bis 100 Gew.-% besitzt.

Das Mittel (III) enthält mindestens eine farbgebende Verbindung (b) und mindestens ein filmbildendes, hydrophiles Polymer (c), wie sie im Detail bereits bei der Beschreibung des ersten Erfindungsgegenstands offenbart wurden.

In einer weiteren bevorzugten Ausführungsform ist eine erfindungsgemäße Mehrkomponenten-Verpackungseinheit dadurch gekennzeichnet, dass das Mittel (III) - bezogen auf das Gesamtgewicht des Mittels (III) - ein oder mehrere farbgebende Verbindungen (b) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

In einer besonders bevorzugten Ausführungsform enthält das Mittel (III) die bereits genannten, die bevorzugten und die besonders bevorzugten filmbildenden, hydrophilen Polymere (c).

Die Mittel (I) und (II) bzw. die Mittel (I), (II) und (III) können in verschiedenen Mengen miteinander vermischt werden. So kann beispielsweise der erste Container 5 g bis 200 g des Mittels (I) enthalten. Der zweite Container kann 5 g bis 200 g des Mittels (II) enthalten. Der dritte Container kann 5 b bis 200 g des Mittels (III) enthalten.

### Weitere Inhaltsstoffe

Die zuvor beschriebenen Mittel, d.h. das anwendungsbereite Mittel des ersten Erfindungsgegenstands, und auch die Mittel (I), (II) und (III) des erfindungsgemäßen Kits des zweiten Erfindungsgegenstands, können ferner auch noch ein oder mehrere optionale Inhaltsstoffe enthalten.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden. Die nichtionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von Keratinmaterialien

Die zuvor beschriebenen Mittel - sowohl das anwendungsbereite Mittel des ersten Erfindungsgegenstandes als auch die Mittel der erfindungsgemäßen Mehrkomponenten-Verpackungseinheit des zweiten Erfindungsgegenstandes - werden in Verfahren zum Färben von keratinischen Materialien, insbesondere zum Färben von menschlichen Haaren, eingesetzt.

Ein dritter Gegenstand der vorliegenden Erfindung ist ein Verfahren zum Färben von keratinischem Material, insbesondere menschlichen Haaren, umfassend die folgenden Schritte in der angegebenen Reihenfolge:
(1) Anwendung eines Vorbehandlungsmittels (V) auf dem keratinischen Material, wobei das Vorbehandlungsmittel (V) in einem wasserhaltigen kosmetischen Träger mindestens eine organische Siliciumverbindung (a) der Formel (I) enthält, wie sie im Detail bereits bei der Beschreibung des ersten Erfindungsgegenstands offenbart wurde,
(2) Anwendung eines Färbemittels (F) auf dem keratinischen Material, wobei das Färbemittel mindestens eine farbgebende Verbindung aus der Gruppe der Pigmente (b) und mindestens ein filmbildendes, hydrophiles Polymer (c) ausgewählt aus der Gruppe aus Polyvinylpyrrolidon (PVP) und den Copolymeren des Polyvinylpyrrolidons enthält, wie sie im Detail bereits bei der Beschreibung des ersten Erfindungsgegenstands offenbart wurden.

Im Rahmen des erfindungsgemäßen Verfahrens werden die Keratinmaterialien, insbesondere die menschlichen Haare, zunächst mit einem Vorbehandlungsmittel (V) behandelt. Im Anschluss daran wird das eigentliche Färbemittel (F) - welches neben dem filmbildenden hydrophilen Polymer den oder die farbgebenden Verbindungen enthält - auf die Keratinmaterialien gegeben.

Bevorzugt enthält das Vorbehandlungsmittel (V) selbst keine Farbstoffe bzw. keine farbgebenden Verbindungen. Kennzeichnend für das Vorbehandlungsmittel (V) ist sein Gehalt an mindestens einer eine reaktiven organische Siliciumverbindung (a) der Formel (I). Das oder die reaktiven organischen Siliciumverbindungen (a) funktionalisieren die Haaroberfläche, sobald sie mit dieser in Kontakt kommen. Auf diesem Wege wird ein erster, noch ungefärbter Film ausgebildet. Im zweiten Schritt des Verfahrens wird nun ein Färbemittel (F) auf die Haare aufgetragen. Während der Anwendung des Färbemittels (F) auf den Keratinmaterialien erfolgt ebenfalls eine Filmbildung auf der - nun bereits funktionalisierten - Haaroberfläche, wobei nun die farbgebenden Verbindungen in den Film eingebettet und auf diese Weise auf den Haaren abgeschieden werden. Der auf diese Weise "in situ" hergestellte Film, in den die farbgebende Verbindung eingebettet ist, zeichnet sich durch eine herausragende Waschechtheit und ein homogenes Farbergebnis aus.

Das Vorbehandlungsmittel (V) stellt das anwendungsbereite Vorbehandlungsmittel (V) dar. Ganz besonders bevorzugt handelt es sich bei dem Vorbehandlungsmittel (V) um die Mischung der Mittel (I) und (II) des erfindungsgemäßen Mehrkomponenten-Verpackungseinheit. Das Vorbehandlungsmittel (V) enthält somit mindestens eine organische Siliciumverbindung (a) der zuvor beschriebenen Formel (I), wobei die Reste für die bereits genannten, die bevorzugten und die besonders bevorzugten Substitutenten stehen.

Weiterhin enthält das Vorbehandlungsmittel (V) Wasser, wobei das Wasser aus dem Mittel (II) des erfindungsgemäßen Kit-of-parts stammt.

In einer ganz besonders bevorzugten Ausführungsform ist ein erfindungsgemäßes Verfahren dadurch gekennzeichnet, dass das Vorbehandlungsmittel (V) vor der Anwendung auf dem keratinischen Material durch Vermischen eines ersten Mittels (I) und eines zweiten Mittels (II) hergestellt wird, wobei
- das Mittel (I) mindestens eine organische Siliciumverbindung (a) der Formel (I) enthält, wie sie bei der Beschreibung des ersten und des zweiten Erfindungsgegenstands, im Detail offenbart wurden und
- das Mittel (II) Wasser enthält.

Es ist bevorzugt, wenn das Vorbehandlungsmittel (V) - bezogen auf das Gesamtgewicht des Vorbehandlungsmittels (V) - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

In einer weiteren ganz besonders bevorzugten Ausführungsform ist ein erfindungsgemäßes Verfahren dadurch gekennzeichnet, dass das Vorbehandlungsmittel (V) - bezogen auf das Gesamtgewicht des Vorbehandlungsmittels - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

Besonders resistente Färbungen konnten bei Anwendung eines alkalisch eingestellten Vorbehandlungsmittels (V) erhalten werden. Bevorzugt besitzt das Vorbehandlungsmittel (V) einen pH-Wert von 7,0 bis 11,5, bevorzugt von 7,5 bis 11,0 und besonders bevorzugt von 8,0 bis 10,5.

In einer weiteren ganz besonders bevorzugten Ausführungsform ist ein erfindungsgemäßes Verfahren dadurch gekennzeichnet, dass das Vorbehandlungsmittel (V) einen pH-Wert von 7,0 bis 11,5, bevorzugt von 7,5 bis 11,0 und besonders bevorzugt von 8,0 bis 10,5 besitzt.

Zur Einstellung dieses alkalischen pH-Wertes enthält das Vorbehandlungsmittel (V) bevorzugt mindestens ein Alkalisierungsmittel, die in einer Menge zugesetzt werden, die die Einstellung des für die jeweilige Haarbehandlung optimalen pH-Wertes gewährleistet. Bei den pH-Werten im Sinne der vorliegenden Erfindung handelt es sich um pH-Werte, die bei einer Temperatur von 22°C gemessen wurden.

Je nach Wahl des gewünschten pH-Wertes und abhängig vom Vorhandensein weiterer Komponenten im erfindungsgemäßen Mittel, wie beispielsweise saurer oder basischer Salze oder Pufferkomponenten, kann die Menge es zugesetzten Alkalisierungsmittel variieren, üblicherweise sind hierfür Mengen von 0,01 bis 15 Gew.-% notwendig.

Als Alkalisierungsmittel kann das Vorbehandlungsmittel (V) beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Vorbehandlungsmittel (V) mindestens ein Alkalisierungsmittel enthält, das bevorzugt ausgewählt ist aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierunsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Im Anschluss an die Anwendung des Vorbehandlungsmittels (V) wird das Färbemittel (F) auf den Keratinmaterialien appliziert. Bei dem Färbemittel (F) handelt es sich um das anwendungsbereite Färbemittel (F).

Das Färbemittel (F) enthält den oder die direktziehenden Farbstoffe (b) und das oder die hydrophoben filmbildenden Polymere (c) in einem kosmetischen Träger, bevorzugt in einem wasserhaltigen kosmetischen Träger.

In einer weiteren ganz besonders bevorzugten Ausführungsform ist ein erfindungsgemäßes Verfahren dadurch gekennzeichnet, dass das Färbemittel (F) - bezogen auf das Gesamtgewicht des Färbemittels (F) - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

Zur Erzeugung von ganz besonders waschechten Färbungen hat es sich weiterhin als ganz besonders bevorzugt herausgestellt, wenn auch das Färbemittel (F) alkalisch eingestellt ist und einen pH-Wert von 7,0 bis 11,5, bevorzugt von 7,5 bis 11,0 und besonders bevorzugt von 8,0 bis 10,5 besitzt.

In einer weiteren ganz besonders bevorzugten Ausführungsform ist ein erfindungsgemäßes Verfahren dadurch gekennzeichnet, dass das Färbemittel (F) einen pH-Wert von 7,0 bis 11,5, bevorzugt von 7,5 bis 11,0 und besonders bevorzugt von 8,0 bis 10,5 besitzt.

Zur Einstellung dieses alkalischen pH-Wertes enthält auch das Färbemittel (F) bevorzugt mindestens ein Alkalisierungsmittel, die in einer Menge zugesetzt werden, die die Einstellung des für die jeweilige Haarbehandlung optimalen pH-Wertes gewährleistet. Bei den pH-Werten im Sinne der vorliegenden Erfindung handelt es sich um pH-Werte, die bei einer Temperatur von 22°C gemessen wurden.

Das Färbemittel (F) kann mindestens ein Alkalisierungsmittel aus der zuvor genannten Gruppe. Besonders bevorzugt enthält das Färbemittel (F) mindestens ein Alkalisierungsmittel enthält, das bevorzugt ausgewählt ist aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel (F) mindestens ein Alkalisierungsmittel enthält, das bevorzugt ausgewählt ist aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

### Verfahrensschritte

Die anwendungstechnischen Eigenschaften der resultierenden Färbung können durch Wahl der optimalen Verfahrensbedingungen noch weiter verbessert werden.

Im Rahmen einer weiteren Ausführungsform ganz besonders bevorzugt ist ein Verfahren, umfassend die folgenden Schritte in der angegebenen Reihenfolge
(1) Anwendung des Vorbehandlungsmittels (V) auf dem keratinischen Material,
(2) Einwirken lassen des Vorbehandlungsmittels (V) für einen Zeitraum von 10 Sekunden bis 10 Minuten, bevorzugt von 10 Sekunden bis 5 Minuten,
(3) gegebenenfalls Ausspülen des Vorbehandlungsmittels (V),
(4) Anwendung des Färbemittels (F) auf dem keratinischen Material,
(5) Einwirken lassen des Färbemittels (F) für einen Zeitraum von 30 Sekunden bis 30 Minuten, bevorzugt von 30 Sekunden bis 10 Minuten, und
(6) gegebenenfalls Anwendung eines Conditioners und
(7) Ausspülen des keratinischen Materials.

In einem Schritt (1) wird zunächst das Vorbehandlungsmittel (V) auf die Keratinmaterialien, insbesondere die menschlichen Haare, appliziert.

Nach dem Auftragen wird das Vorbehandlungsmittel (V) auf die Keratinmaterialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

In einer weiteren bevorzugten Ausführungsform ist ein erfindungsgemäßes Verfahren gekennzeichnet durch das
(2) Einwirken lassen des Vorbehandlungsmittels (V) für einen Zeitraum von 10 Sekunden bis 10 Minuten, bevorzugt 10 Sekunden bis 5 Minuten und ganz besonders bevorzugt von 30 Sekunden bis 2 Minuten auf die Keratinmaterialien.

Im Rahmen einer bevorzugten Ausführungsform des erfindungsgemäßen Verfahrens kann das Vorbehandlungsmittel (V) nun von den Keratinmaterialien ausgespült werden, bevor das Färbemittel (F) im nachfolgenden Schritt auf die Haare appliziert wird.

In einer weiteren Ausführungsform ganz besonders bevorzugt ist ein Verfahren, umfassend die folgenden Schritte in der angegebenen Reihenfolge
(1) Anwendung des Vorbehandlungsmittels (V) auf dem keratinischen Material,
(2) Einwirken lassen des Vorbehandlungsmittels (V) für einen Zeitraum von 10 Sekunden bis 10 Minuten, bevorzugt von 10 Sekunden bis 5 Minuten,
(3) Ausspülen des Vorbehandlungsmittels (V),
(4) Anwendung des Färbemittels (F) auf dem keratinischen Material,
(5) Einwirken lassen des Färbemittels (F) für einen Zeitraum von 30 Sekunden bis 30 Minuten, bevorzugt von 30 Sekunden bis 10 Minuten, und
(6) gegebenenfalls Anwendung eines Conditioners und
(7) Ausspülen des keratinischen Materials.

Färbungen mit ebenfalls guten Waschechtheiten wurden erhalten, wenn das Färbemittel (F) auf die Keratinmaterialien appliziert wurde, die noch mit dem Vorbehandlungsmittel (V) beaufschlagt waren.

In einer weiteren Ausführungsform ganz besonders bevorzugt ist ein Verfahren, umfassend die folgenden Schritte in der angegebenen Reihenfolge
(1) Anwendung des Vorbehandlungsmittels (V) auf dem keratinischen Material,
(2) Einwirken lassen des Vorbehandlungsmittels (V) für einen Zeitraum von 10 Sekunden bis 10 Minuten, bevorzugt von 10 Sekunden bis 5 Minuten,
(3) kein Ausspülen des Vorbehandlungsmittels (V),
(4) Anwendung des Färbemittels (F) auf dem keratinischen Material,
(5) Einwirken lassen des Färbemittels (F) für einen Zeitraum von 30 Sekunden bis 30 Minuten, bevorzugt von 30 Sekunden bis 10 Minuten, und
(6) gegebenenfalls Anwendung eines Conditioners und
(7) Ausspülen des keratinischen Materials.

In Schritt (4) wird nun das Färbemittel (F) auf die Keratinmaterialien appliziert. Nach dem Auftragen wird nun das Färbemittel (F) auf die Haare einwirken gelassen.

Das erfindungsgemäße Verfahren erlaubt selbst bei kurzer Einwirkzeit des Färbemittels (F) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In einer weiteren bevorzugten Ausführungsform ist ein erfindungsgemäßes Verfahren gekennzeichnet durch das
(5) Einwirken lassen des Färbemittels (F) für einen Zeitraum von 10 Sekunden bis 10 Minuten, bevorzugt von 20 Sekunden bis 5 Minuten und ganz besonders bevorzugt von 30 Sekunden bis 3 Minuten auf die Haare.

In einer weiteren Ausführungsform ganz besonders bevorzugt ist ein Verfahren, umfassend die folgenden Schritte in der angegebenen Reihenfolge
(1) Anwendung des Vorbehandlungsmittels (V) auf dem keratinischen Material,
(2) Einwirken lassen des Vorbehandlungsmittels (V) für einen Zeitraum von 10 Sekunden bis 10 Minuten, bevorzugt von 10 Sekunden bis 5 Minuten,
(3) Ausspülen des Vorbehandlungsmittels (V),
(4) Anwendung des Färbemittels (F) auf dem keratinischen Material,
(5) Einwirken lassen des Färbemittels (F) für einen Zeitraum von 10 Sekunden bis 10 Minuten, bevorzugt von 20 Sekunden bis 5 Minuten und ganz besonders bevorzugt von 30 Sekunden bis 3 Minuten auf die Haare, und
(6) gegebenenfalls Anwendung eines Conditioners und
(7) Ausspülen des keratinischen Materials.

Nach dem Einwirken des Färbemittel (F) kann nun optional ein Conditioner zur Anwendung kommen.

In einer weiteren Ausführungsform ganz besonders bevorzugt ist ein Verfahren, umfassend die folgenden Schritte in der angegebenen Reihenfolge
(1) Anwendung des Vorbehandlungsmittels (V) auf dem keratinischen Material,
(2) Einwirken lassen des Vorbehandlungsmittels (V) für einen Zeitraum von 10 Sekunden bis 10 Minuten, bevorzugt von 10 Sekunden bis 5 Minuten,
(3) Ausspülen des Vorbehandlungsmittels (V),
(4) Anwendung des Färbemittels (F) auf dem keratinischen Material,
(5) Einwirken lassen des Färbemittels (F) für einen Zeitraum von 10 Sekunden bis 10 Minuten, bevorzugt von 20 Sekunden bis 5 Minuten und ganz besonders bevorzugt von 30 Sekunden bis 3 Minuten auf die Haare, und
(6) Anwendung eines Conditioners und
(7) Ausspülen des keratinischen Materials.

Bevorzugt enthält der Conditioner mindestens ein kationisches und/oder nichtionisches Tensid.

Überraschenderweise hat sich hierbei herausgestellt, dass der Einsatz des Conditioners - insbesondere wenn dieser mindestens ein kationisches Tensid enthält - die Echtheit der erhaltenen Färbungen noch weiter zu verbessern mag und das Farbergebnis zusätzlich intensiviert.

In einer weiteren bevorzugten Ausführungsform ist ein erfindungsgemäßes Verfahren dadurch gekennzeichnet, dass der Conditioner mindestens ein kationisches und/oder nichtionisches Tensid enthält.

In einer weiteren ganz besonders bevorzugten Ausführungsform ist ein erfindungsgemäßes Verfahren dadurch gekennzeichnet, dass der Conditioner mindestens ein kationisches Tensid enthält.

Um eine möglichst homogene und widerstandsfähige Färbung zu erhalten, hat es sich als besonders bevorzugt herausgestellt, wenn zwischen der Anwendung des Vorbehandlungsmittels (V) und der Anwendung des Färbemittels (F) ein Zeitraum von maximal 48 Stunden, bevorzugt maximal 24 Stunden, weiter bevorzugt von maximal 12 Stunden und ganz besonders bevorzugt von maximal 6 Stunden liegt.

In einer weiteren bevorzugten Ausführungsform ist ein erfindungsgemäßes Verfahren dadurch gekennzeichnet, dass das Vorbehandlungsmittel (V) und das Färbemittel (F) innerhalb eines Zeitraums von maximal 48 Stunden, bevorzugt maximal 24 Stunden, weiter bevorzugt von maximal 12 Stunden und ganz besonders bevorzugt von maximal 6 Stunden auf die Haare appliziert werden.

Betreffend die weiteren bevorzugten Ausführungsformen der erfindungsgemäßen Mehrkomponenten-Verpackungseinheit und der erfindungsgemäßen Verfahren gilt *mutatis mutantis* das zum erfindungsgemäßen Mittel gesagte.

### Beispiele

### 1. Formulierungen

Es wurden die folgenden Formulierungen hergestellt (sofern nichts anderes angegeben ist, sind alle Angaben in Gew.-%)

### Vorbehandlungsmittel (V)

| Mittel (I) | (I) |
|---|---|
| (3-Aminopropyl)triethoxysilan | 0,3 g |

| Mittel (II) | (II) |
|---|---|
| Natriumhydroxid | ad pH 10,0 |
| Wasser | ad 100 Gew.-% |

Durch Vermischen von 0,3 g des Mittels (I) und 100 g des Mittels (II) wurde das anwendungsbereite Vorbehandlungsmittel (V) hergestellt. Hierbei wurden die Mittel (I) und (II) für 3 Minuten miteinander verschüttelt. Dann wurde das Vorbehandlungsmittel (V) für ca. 5 Minuten stehen gelassen. Der pH-Wert des anwendungsbereiten Vorbehandlungsmittels (V) lag bei ca. 10.

### Färbemittel (F)

| Mittel (III) | V | E | V | E |
|---|---|---|---|---|
| | F1 | F2 | F3 | F4 |
| Colorona Bronze (Merck, Mica, CI77491, Iron oxides, CI77019) | 2,0 | 2,0 | --- | --- |
| Unipure Red LC 3071 (Sensient, Aluminium hydroxide, CI 15850) | --- | --- | 3,0 | 3,0 |
| PVP K 30 (Ashland, ISP, Polyvinylpyrrolidone) | --- | 9,0 | --- | 9,0 |
| Dermacryl 79 (Akzo Nobel, Acrylates/Octylacrylamide Copolymer, CAS-Nr. 129702-02-9) | 9,0 | --- | 9,0 | --- |
| Ammoniak (25 %ige wässrige Lösung) | ad pH 10 | ad pH 10 | ad pH 10 | ad pH 10 |
| Wasser | ad 100 | ad 100 | ad 100 | ad 100 |

| | | | | |
|---|---|---|---|---|
| V = Vergleich E = erfindungsgemäß | | | | |

### Conditioner (C)

| | |
|---|---|
| | |
| Cetearylalkohol | 5,0 |
| Ceteareth-20 | 0,3 |
| Isopropylmyristat | 0,8 |
| Stearamidopropyldimethylamin | 0,4 |
| Quaternium-87 | 3,0 |
| Propylenglycol | 1,0 |
| Zitronensäure | 0,95 |
| Glycerylstearat | 0,3 |
| Wasser | Ad 100 |

### 2. Anwendung

Jeweils eine Haarsträhne (Kerling, Euronaturhaar weiß) wurde in das Vorbehandlungsmittel (V) getaucht und für 1 Minuten darin belassen. Danach wurde überflüssiges Vorbehandlungsmittel von jeder Haarsträhne gestreift. Jede Haarsträhne wurde mit Wasser ausgewaschen. Überschüssiges Wasser wurde von jeder Haarsträhne gestreift.

Im Anschluss daran wurden die Haarsträhnen jeweils in eines der Färbemittel (F) getaucht und für 1 Minute darin belassen.

Danach wurden die die Haarsträhnen jeweils mit einer kleinen Menge des Conditioners benetzt und anschließend mit Wasser ausgewaschen und getrocknet. Im Anschluss daran wurden die Strähen visuell bewertet.

| Bsp | 1 | 2 | 3 | 4 |
|---|---|---|---|---|
| Vorbehandlungsmittel (V) | (V) | (V) | (V) | (V) |
| Färbemittel (F) | (F1) Colorona Bronze + Dermacryl 79 | (F2) Colorona Bronze + PVP K30 | (F3) Unipure Red LC 3071 + Dermacryl 79 | (F4) Unipure Red LC 3071 + PVP K 30 |

| Conditioner (C) | (C) | (C) | (C) | (C) |
|---|---|---|---|---|
| Färbung Farbintensität Haargefühl | bronzegelb +++ | goldgelb +++ | rot +++ | rot +++ |

### 3. Messung der Lagerstabiltät

Von jeder der Farbcremes F1 bis F4 wurden 100 g in einen Glasbehälter abgefüllt. Dann wurde der Glasbehälter verschlossen. Anschließend wurde jeder Behälter bei Raumtemperatur für 4 Wochen gelagert. Nach diesem Lagerzeitraum wurde die Stabilität der Formulierung optisch bewertet.

| | V | E | V | E |
|---|---|---|---|---|
| | F1 | F2 | F3 | F4 |
| | (F1) Colorona Bronze + Dermacryl 79 | (F2) Colorona Bronze + PVP K30 | (F3) Unipure Red LC 3071 + Dermacryl 79 | (F4) Unipure Red LC 3071 + PVP K 30 |
| Lagerung 4 Wochen, Raumtemperatur | Pigmente lagern sich am Boden des Gefäßes ab, Separation sichtbar | fein dispergiert | Pigmente lagern sich am Boden des Gefäßes ab, Separation sichtbar | fein dispergiert |

## Patentansprüche

1. Mittel zum Färben von keratinischem Material, insbesondere menschlichen Haaren, enthaltend in einem kosmetischen Träger
(a) mindestens eine organische Siliciumverbindung der Formel (I),
R₁R₂N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht, und
(b) mindestens eine farbgebende Verbindung aus der Gruppe der Pigmente, und
(c) mindestens ein filmbildendes, hydrophiles Polymer ausgewählt aus der Gruppe aus Polyvinylpyrrolidon (PVP) und den Copolymeren des Polyvinylpyrrolidons.

2. Mittel nach Anspruch 1, **dadurch gekennzeichnet, dass** es (a) mindestens eine organische Siliciumverbindung der Formel (I) enthält,
R₁R₂N-L-Si(OR₃)ₐ(R₄)_{b} (I),
wobei
- R₁, R₂ beide für ein Wasserstoffatom stehen, und
- L für eine Propylengruppe (-CH₂-CH₂-CH₂-) oder für eine Ethylengruppe (-CH₂-CH₂-), steht.

3. Mittel nach einem der Ansprüche 1 bis 2, **dadurch gekennzeichnet, dass** es (a) mindestens eine organische Siliciumverbindung der Formel (I) enthält,
wobei
- R₃, R₄ unabhängig voneinander für eine Methylgruppe oder für eine Ethylgruppe stehen und
- a für die Zahl 3 steht und
- b für die Zahl 0 steht.

4. Mittel nach einem der Ansprüche 1 bis 3, **dadurch gekennzeichnet, dass** es (a) mindestens eine organische Siliciumverbindung der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan und/oder
- 1-(2-Aminoethyl)silantriol.

5. Mittel nach einem der Ansprüche 1 bis 4, **dadurch gekennzeichnet, dass** es
(b) mindestens eine farbgebende Verbindung aus der Gruppe der anorganischen und/oder organischen Pigmente enthält.

6. Mittel nach einem der Ansprüche 1 bis 5, **dadurch gekennzeichnet, dass** es
(b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

7. Mittel nach einem der Ansprüche 1 bis 6, **dadurch gekennzeichnet, dass** es
(b) mindestens eine farbgebende Verbindung aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

8. Mittel nach einem der Ansprüche 1 bis 7, **dadurch gekennzeichnet, dass** es
(b) mindestens eine farbgebende Verbindungen aus der Gruppe der organischen Pigmente enthält, die ausgewählt ist aus der Gruppe aus Carmin, Chinacridon, Phthalocyanin, Sorgho, CI 42090, CI 69800, CI 69825, CI 73000, CI 74100, CI 74160, CI 11680, CI 11710, CI 15985, CI 19140, CI 20040, CI 21100, CI 21108, CI 47000, CI 47005, CI 61565, CI 61570, CI 74260, CI 11725, CI 15510, CI 45370, CI 71105, CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

9. Mittel nach einem der Ansprüche 1 bis 8, **dadurch gekennzeichnet, dass** es (c) mindestens ein filmbildendes, hydrophiles Polymer enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

10. Mittel nach einem der Ansprüche 1 bis 9, **dadurch gekennzeichnet, dass** es (c) mindestens ein nichtionisches, filmbildendes, hydrophiles Polymer enthält.

11. Mittel nach einem der Ansprüche 1 bis 10, **dadurch gekennzeichnet, dass** es - bezogen auf sein Gesamtgewicht - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

12. Mehrkomponenten-Verpackungseinheit (Kit-of-parts) zum Färben von keratinischem Material, insbesondere menschlichen Haaren, welche getrennt voneinander konfektioniert
- einen ersten Container enthaltend ein kosmetisches Mittel (I) und
- einen zweiten Container enthaltend ein kosmetisches Mittel (II) und
- einen dritten Container enthaltend ein kosmetisches Mittel (III) umfasst,
wobei
- das Mittel (I) mindestens eine organische Siliciumverbindung (a) enthält, wie sie in einem der Ansprüche 1, 2, 3 und/oder 4 definiert ist,
- das Mittel (II) Wasser enthält und
- das Mittel (III) mindestens eine farbgebende Verbindung aus der Gruppe der Pigmente (b) und mindestens ein filmbildendes, hydrophiles Polymer (c) enthält, wie sie ein einem der Ansprüche 1, 5, 6, 7, 9 und/oder 10 definiert sind.

13. Verfahren zum Färben von keratinischem Material, insbesondere menschlichen Haaren, umfassend die folgenden Schritte in der angegebenen Reihenfolge:
(1) Anwendung eines Vorbehandlungsmittels (V) auf dem keratinischen Material, wobei das Vorbehandlungsmittel (V) in einem wasserhaltigen kosmetischen Träger mindestens eine organische Siliciumverbindung (a) enthält, wie sie in einem der Ansprüche 1, 2, 3 und/oder 4 definiert ist, und
(2) Anwendung eines Färbemittels (F) auf dem keratinischen Material, wobei das Färbemittel mindestens eine farbgebende Verbindung aus der Gruppe der Pigmente (b) und mindestens ein filmbildendes, hydrophiles Polymer (c) enthält, wie sie in den Ansprüchen 1, 5, 6, 7, 9 und/oder 10 definiert sind.

14. Verfahren nach Anspruch 13, **dadurch gekennzeichnet, dass** das Vorbehandlungsmittel (V) vor der Anwendung auf dem keratinischen Material durch Vermischen eines ersten Mittels (I) und eines zweiten Mittels (II) hergestellt wird, wobei
- das Mittel (I) mindestens eine organische Siliciumverbindung (a) enthält, wie sie in einem der Ansprüche 1, 2, 3 und/oder 4 definiert ist, und
- das Mittel (II) Wasser enthält.

15. Verfahren nach einem der Ansprüche 13 bis 14, **dadurch gekennzeichnet, dass** das Vorbehandlungsmittel (V) - bezogen auf das Gesamtgewicht des Vorbehandlungsmittels - einen Wassergehalt von 15 bis 95 Gew.-%, bevorzugt von 20 bis 95 Gew.-%, weiter bevorzugt von 25 bis 95 Gew.-%, noch weiter bevorzugt von 30 bis 95 Gew.-% und ganz besonders bevorzugt von 45 bis 95 Gew.-% besitzt.

16. Verfahren nach einem der Ansprüche 13 bis 15, umfassend die folgenden Schritte in der angegebenen Reihenfolge
(1) Anwendung des Vorbehandlungsmittels (V) auf dem keratinischen Material,
(2) Einwirken lassen des Vorbehandlungsmittels (V) für einen Zeitraum von 10 Sekunden bis 10 Minuten, bevorzugt von 10 Sekunden bis 5 Minuten,
(3) gegebenenfalls Ausspülen des Vorbehandlungsmittels (V),
(4) Anwendung des Färbemittels (F) auf dem keratinischen Material,
(5) Einwirken lassen des Färbemittels (F) für einen Zeitraum von 30 Sekunden bis 30 Minuten, bevorzugt von 30 Sekunden bis 10 Minuten, und
(6) gegebenenfalls Anwendung eines Conditioners und
(7) Ausspülen des keratinischen Materials.

17. Verfahren nach Anspruch 16, **dadurch gekennzeichnet, dass** der Conditioner mindestens ein kationisches und/oder nichtionisches Tensid enthält.

18. Verfahren nach einem der Ansprüche 13 bis 17, **dadurch gekennzeichnet, dass** das Vorbehandlungsmittel (V) und das Färbemittel (F) innerhalb eines Zeitraums von maximal 48 Stunden, bevorzugt maximal 24 Stunden, weiter bevorzugt von maximal 12 Stunden und ganz besonders bevorzugt von maximal 6 Stunden auf den Haaren angewendet werden.

## Claims

1. An agent for dyeing keratinous material, in particular human hair, containing, in a cosmetic carrier,
(a) at least one organosilicon compound of formula (I),
R₁R₂N-L-Si(OR₃)ₐ(R₄)_{b} (I),
where
- R₁, R₂ both represent a hydrogen atom, and
- L represents a linear, divalent C₁-C₆ alkylene group,
- R₃ represents a hydrogen atom or a C₁-C₆ alkyl group,
- R₄ represents a C₁-C₆ alkyl group,
- a represents an integer from 1 to 3, and
- b represents the integer 3 - a, and
(b) at least one coloring compound from the group of pigments, and
(c) at least one film-forming, hydrophilic polymer selected from the group of polyvinylpyrrolidone (PVP) and the copolymers of polyvinylpyrrolidone.

2. The agent according to claim 1, **characterized in that** it contains (a) at least one organosilicon compound of formula (I),
R₁R₂N-L-Si(OR₃)ₐ(R₄)_{b} (I),
where
- R₁, R₂ both represent a hydrogen atom, and
- L represents a propylene group (-CH₂-CH₂-CH₂-) or an ethylene group (-CH₂-CH₂-).

3. The agent according to one of claims 1 to 2, **characterized in that** it contains (a) at least one organosilicon compound of formula (I),
where
- R₃, R₄ represent, independently of one another, a methyl group or an ethyl group, and
- a represents the number 3, and
- b represents the number 0.

4. The agent according to one of claims 1 to 3, **characterized in that** it contains (a) at least one organosilicon compound of formula (I), selected from the group of
- (3-aminopropyl)triethoxysilane,
- (3-aminopropyl)trimethoxysilane,
- 1-(3-aminopropyl)silanetriol
- (2-aminoethyl)triethoxysilane,
- (2-aminoethyl)trimethoxysilane, and/or
- 1-(2-aminoethyl)silanetriol.

5. The agent according to one of claims 1 to 4, **characterized in that** it contains (b) at least one coloring compound from the group of inorganic and/or organic pigments.

6. The agent according to one of claims 1 to 5, **characterized in that** it contains (b) at least one coloring compound from the group of pigments selected from the group of colored metal oxides, metal hydroxides, metal oxide hydrates, silicates, metal sulfides, complex metal cyanides, metal sulfates, bronze pigments and/or from mica-based colored pigments which are coated with at least one metal oxide and/or a metal oxychloride.

7. The agent according to one of claims 1 to 6, **characterized in that** it contains (b) at least one coloring compound from the group of pigments selected from mica-based pigments which are coated with one or more metal oxides from the group of titanium dioxide (CI 77891), black iron oxide (CI 77499), yellow iron oxide (CI 77492), red and/or brown iron oxide (CI 77491, CI 77499), manganese violet (CI 77742), ultramarine (sodium aluminum sulfosilicates, CI 77007, Pigment Blue 29), chromium oxide hydrate (CI 77289), chromium oxide (CI 77288), and/or iron blue (ferric ferrocyanide, CI 77510).

8. The agent according to one of claims 1 to 7, **characterized in that** it contains (b) at least one coloring compound from the group of organic pigments selected from the group of carmine, quinacridone, phthalocyanine, sorghum, CI 42090, CI 69800, CI 69825, CI 73000, CI 74100, CI 74160, CI 11680, CI 11710, CI 15985, CI 19140, CI 20040, CI 21100, CI 21108, CI 47000, CI 47005, CI 61565, CI 61570, CI 74260, CI 11725, CI 15510, CI 45370, CI 71105, CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

9. The agent according to one of claims 1 to 8, **characterized in that** it contains (c) at least one film-forming, hydrophilic polymer selected from the group of polyvinylpyrrolidone (PVP), vinylpyrrolidone/vinyl acetate copolymers, vinylpyrrolidone/styrene copolymers, vinylpyrrolidone/ethylene copolymers, vinylpyrrolidone/propylene copolymers, vinylpyrrolidone/vinylcaprolactam copolymers, vinylpyrrolidone/vinylformamide copolymers and/or vinylpyrrolidone/vinyl alcohol copolymers, explicitly very particularly preferably polyvinylpyrrolidone (PVP).

10. The agent according to one of claims 1 to 9, **characterized in that** it contains (c) at least one non-ionic, film-forming, hydrophilic polymer.

11. The agent according to one of claims 1 to 10, **characterized in that** it has, based on its total weight, a water content of 15 to 95 wt.%, preferably of 20 to 95 wt.%, more preferably of 25 to 95 wt.%, even more preferably of 30 to 95 wt.% and very particularly preferably of 45 to 95 wt.%.

12. A multi-component packaging unit (kit-of-parts) for dyeing keratinous material, in particular human hair, comprising, packaged separately from one another,
- a first container containing a cosmetic agent (I), and
- a second container containing a cosmetic agent (II), and
- a third container containing a cosmetic agent (III),
wherein
- the agent (I) contains at least one organosilicon compound (a) as defined in one of claims 1, 2, 3 and/or 4,
- the agent (II) contains water, and
- the agent (III) contains at least one coloring compound from the group of pigments (b) and at least one film-forming, hydrophilic polymer (c), as are defined in one of claims 1, 5, 6, 7, 9 and/or 10.

13. A method for dyeing keratinous material, in particular human hair, comprising the following steps in the specified order:
(1) applying a pretreatment agent (V) to the keratinous material, wherein the pretreatment agent (V) contains at least one organosilicon compound (a), as defined in one of claims 1, 2, 3 and/or 4, in a water-containing cosmetic carrier, and
(2) applying a coloring agent (F) to the keratinous material, wherein the coloring agent contains at least one coloring compound from the group of pigments (b) and at least one film-forming, hydrophilic polymer (c), as are defined in claims 1, 5, 6, 7, 9 and/or 10.

14. The method according to claim 13, **characterized in that** the pretreatment agent (V) is prepared before application to the keratinous material by mixing a first agent (I) and a second agent (II),
- the agent (I) containing at least one organosilicon compound (a) as defined in one of claims 1, 2, 3 and/or 4, and
- the agent (II) containing water.

15. The method according to one of claims 13 to 14, **characterized in that** the pretreatment agent (V) has, based on the total weight of the pretreatment agent, a water content of 15 to 95 wt.%, preferably of 20 to 95 wt.%, more preferably of 25 to 95 wt.%, even more preferably of 30 to 95 wt.%, and very particularly preferably of 45 to 95 wt.%.

16. The method according to one of claims 13 to 15, comprising the following steps in the specified order
(1) applying the pretreatment agent (v) to the keratinous material,
(2) allowing the pretreatment agent (V) to act for a period of 10 seconds to 10 minutes, preferably 10 seconds to 5 minutes,
(3) optionally rinsing out the pretreatment agent (V),
(4) applying the coloring agent (F) to the keratinous material,
(5) allowing the coloring agent (F) to act for a period of 30 seconds to 30 minutes, preferably 30 seconds to 10 minutes, and
(6) optionally applying a conditioner, and
(7) rinsing the keratinous material.

17. The method according to claim 16, **characterized in that** the conditioner contains at least one cationic and/or non-ionic surfactant.

18. The method according to one of claims 13 to 17, **characterized in that** the pretreatment agent (V) and the coloring agent (F) are applied to the hair within a period of at most 48 hours, preferably of at most 24 hours, more preferably of at most 12 hours and very particularly preferably of at most 6 hours.

## Revendications

1. Agent permettant de colorer de la matière kératinique, en particulier des cheveux humains, contenant dans un support cosmétique
(a) au moins un composé organique de silicium de formule (I),
R₁R₂N-L-Si(OR₃)ₐ(R₄)_{b} (I),
où
- R₁, R₂ représentent tous deux un atome d'hydrogène, et
- L représente un groupe alkylène en C₁-C₆ divalent linéaire,
- R₃ représente un atome d'hydrogène ou un groupe alkyle en C₁-C₆,
- R₄ représente un groupe alkyle en C₁-C₆,
- a, représente un nombre entier allant de 1 à 3, et
- b représente le nombre entier 3 - a, et
(b) au moins un composé colorant du groupe des pigments, et
(c) au moins un polymère hydrophile filmogène choisi dans le groupe constitué de polyvinylpyrrolidone (PVP) et copolymères de la polyvinylpyrrolidone.

2. Agent selon la revendication 1, **caractérisé en ce qu'il** (a) contient au moins un composé organique de silicium de formule (I),
R₁R₂N-L-Si(OR₃)ₐ(R₄)_{b} (I),
où
- R₁, R₂ représentent tous deux un atome d'hydrogène, et
- L représente un groupe propylène (-CH₂-CH₂-CH₂-) ou un groupe éthylène (-CH₂-CH₂-).

3. Agent selon l'une des revendications 1 à 2, **caractérisé en ce qu'**il (a) contient au moins un composé organique de silicium de formule (I),
où
- R₃, R₄ représentent, indépendamment l'un de l'autre, un groupe méthyle ou un groupe éthyle et
- a représente le nombre 3 et
- b représente le nombre 0.

4. Agent selon l'une des revendications 1 à 3, **caractérisé en ce qu'**il (a) contient au moins un composé organique de silicium de formule (II) qui est choisi dans le groupe constitué de
- (3-aminopropyl)triéthoxysilane
- (3-aminopropyl)triméthoxysilane
- 1-(3-aminopropyl)silanetriol
- (2-aminoéthyl)triéthoxysilane
- (2-aminoéthyl)triméthoxysilane et/ou
- 1-(2-aminoéthyl)silanetriol.

5. Agent selon l'une des revendications 1 à 4, **caractérisé en ce qu'**il (b) contient au moins un composé colorant du groupe des pigments inorganiques et/ou organiques.

6. Agent selon l'une des revendications 1 à 5, **caractérisé en ce qu'**il (b) contient au moins un composé colorant du groupe des pigments, lequel composé colorant est choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica recouverts d'au moins un oxyde métallique et/ou oxychlorure métallique.

7. Agent selon l'une des revendications 1 à 6, **caractérisé en ce qu'**il (b) contient au moins un composé colorant du groupe des pigments, lequel composé colorant est choisi parmi les pigments à base de mica recouverts d'un ou de plusieurs oxydes métalliques du groupe constitué de dioxyde de titane (CI 77891), oxyde de fer noir (CI 77499), oxyde de fer jaune (CI 77492), oxyde de fer rouge et/ou brun (CI 77491, CI 77499), violet de manganèse (CI 77742), bleu d'outremer (sulfosilicates de sodium et d'aluminium, CI 77007, Pigment Blue 29), oxyde de chrome hydraté (CI 77289), oxyde de chrome (CI 77288) et/ou bleu de Prusse (ferrocyanure ferrique, CI 77510).

8. Agent selon l'une des revendications 1 à 7, **caractérisé en ce qu'**il (b) contient au moins un composé colorant du groupe des pigments organiques, lequel composé colorant est choisi dans le groupe constitué de carmin, quinacridone, phtalocyanine, sorgho, CI 42090, CI 69800, CI 69825, CI 73000, CI 74100, CI 74160, CI 11680, CI 11710, CI 15985, CI 19140, CI 20040, CI 21100, CI 21108, CI 47000, CI 47005, CI 61565, CI 61570, CI 74260, CI 11725, CI 15510, CI 45370, CI 71105, CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

9. Agent selon l'une des revendications 1 à 8, **caractérisé en ce qu'**il (c) contient au moins un polymère hydrophile filmogène qui est choisi dans le groupe constitué de polyvinylpyrrolidone (PVP), copolymères de vinylpyrrolidone/acétate de vinyle, copolymères de vinylpyrrolidone/styrène, copolymères de vinylpyrrolidone/éthylène, copolymères de vinylpyrrolidone/propylène, copolymères de vinylpyrrolidone/vinylcaprolactame, copolymères de vinylpyrrolidone/vinylformamide et/ou copolymères de vinylpyrrolidone/alcool vinylique, de manière explicite et tout particulièrement préférée est la polyvinylpyrrolidone (PVP).

10. Agent selon l'une des revendications 1 à 9, **caractérisé en ce qu'**il (c) contient au moins un polymère hydrophile, filmogène et non ionique.

11. Agent selon l'une des revendications 1 à 10, **caractérisé en ce qu'**il contient, par rapport à son poids total, une teneur en eau allant de 15 à 95 % en poids, de préférence de 20 à 95 % en poids, plus préférablement de 25 à 95 % en poids, encore plus préférablement de 30 à 95 % en poids et de manière tout particulièrement préférée de 45 à 95 % en poids.

12. Unité de conditionnement à plusieurs composants (kit de pièces) pour la coloration de matière kératinique, en particulier de cheveux humains, comprenant, conditionnés séparément les uns des autres,
- un premier récipient contenant un agent cosmétique (I) et
- un deuxième récipient contenant un agent cosmétique (II) et
- un troisième récipient contenant un agent cosmétique (III),
dans laquelle
- l'agent (I) contient au moins un composé organique de silicium (a) tel que défini dans l'une des revendications 1, 2, 3 et/ou 4,
- l'agent (II) contient de l'eau et
- l'agent (III) contient au moins un composé colorant du groupe des pigments (b) et au moins un polymère hydrophile filmogène (c) tels que définis dans l'une des revendications 1, 5, 6, 7, 9 et/ou 10.

13. Procédé permettant de colorer de la matière kératinique, en particulier des cheveux humains, comprenant les étapes suivantes dans l'ordre indiqué :
(1) application d'un agent de prétraitement (V) sur la matière kératinique, dans lequel l'agent de prétraitement (V) contient, dans un support cosmétique contenant de l'eau, au moins un composé organique de silicium (a) tel que défini dans l'une des revendications 1, 2, 3 et/ou 4, et
(2) application d'un agent de coloration (F) sur la matière kératinique, dans lequel l'agent de coloration contient au moins un composé colorant du groupe des pigments (b) et au moins un polymère hydrophile filmogène (c) tels que définis dans les revendications 1, 5, 6, 7, 9 et/ou 10.

14. Procédé selon la revendication 13, **caractérisé en ce que** l'agent de prétraitement (V) est préparé, avant application sur la matière kératinique, par mélange d'un premier agent (I) et d'un deuxième agent (II), dans lequel
- l'agent (I) contient au moins un composé organique de silicium (a) tel que défini dans l'une des revendications 1, 2, 3 et/ou 4, et
- l'agent (II) contient de l'eau.

15. Procédé selon l'une des revendications 13 à 14, **caractérisé en ce que** l'agent de prétraitement (V) contient, par rapport au poids total de l'agent de prétraitement, une teneur en eau allant de 15 à 95 % en poids, de préférence de 20 à 95 % en poids, plus préférablement de 25 à 95 % en poids, encore plus préférablement de 30 à 95 % en poids et de manière tout particulièrement préférée de 45 à 95 % en poids.

16. Procédé selon l'une des revendications 13 à 15, comprenant les étapes suivantes dans l'ordre indiqué
(1) application de l'agent de prétraitement (V) sur la matière kératinique,
(2) attente de l'action de l'agent de prétraitement (V) pendant une période allant de 10 secondes à 10 minutes, de préférence de 10 secondes à 5 minutes,
(3) le cas échéant, élimination par rinçage de l'agent de prétraitement (V),
(4) application de l'agent de coloration (F) sur la matière kératinique,
(5) attente de l'action de l'agent de coloration (F) pendant une période allant de 30 secondes à 30 minutes, de préférence de 30 secondes à 10 minutes, et
(6) éventuellement, application d'un après-shampooing et
(7) élimination par rinçage de la matière kératinique.

17. Procédé selon la revendication 16, **caractérisé en ce que** l'après-shampooing contient au moins un tensioactif cationique et/ou non ionique.

18. Procédé selon l'une des revendications 13 à 17, **caractérisé en ce que** l'agent de prétraitement (V) et l'agent de coloration (F) sont appliqués sur les cheveux pendant une période d'au maximum de 48 heures, de préférence d'au maximum 24 heures, plus préférablement d'au maximum 12 heures et de manière tout particulièrement préférée d'au maximum 6 heures.
